# EUROPEAN PATENT APPLICATION

(11) **EP 4 286 849 A1**
(43) Date of publication of application: **06.12.2023**
(21) Application number: 22305813.2
(22) Date of filing: 03.06.2022
(51) Int. Cl.: G01N 33/543, G01N 33/50, G01N 33/53, G01N 33/551

(54) **METHOD FOR QUANTITATIVE MEASUREMENT OF REDUCING SUGARS, AND FLUORESCENT PROBES USED THEREIN**

(71) Applicant: Wainvam-E, 56270 Ploemeur (FR)
(72) Inventor: LE GUEVELLO, Morgane, 56300 Malguénac (FR); RANI, Dipti, 56100 Lorient (FR)
(74) Representative: Novagraaf Technologies

(57) **Abstract**

The present invention relates to a method for quantitative measurement of reducing sugars in a sample, comprising the steps of:
(1) Functionalizing nanodiamonds having NV centers with ligands able to bind specifically to said reducing sugars,
(2) Bringing said functionalized nanodiamonds into contact with said reducing sugars, under conditions allowing binding between said nanodiamond and said reducing sugars,
(3) Illuminating the NV centers with green light, and
(4) Measuring red fluorescence emitted by the NV centers,
wherein the amount of red fluorescence emitted by the NV centers is proportional to the amount of nanodiamonds bound to the reducing sugars.

The present invention also relates to a carboxylated nanodiamond having NV centers, which is functionalized with aptamers or with polyclonal antibodies that are able to bind specifically to said reducing sugars, and to a device comprising a processor and memory, said memory comprising code that, when executed by said processor, causes the device to perform the method of the invention.

The present invention further relates to the use of the method as defined above, or of a functionalized nanodiamond as defined above, for quantitative measurement of reducing sugars in the food industry.

Finally, the invention relates to a method for preparing a carboxylated nanodiamond having NV centers, which is functionalized with aptamers that are able to bind specifically to said reducing sugars.

## Description

### Technical field

The present invention refers to a method for quantitative measurement of reducing sugars in a sample, to functionalized nanodiamonds having Nitrogen-Vacancy (NV) centers, and to the use of the method or of the functionalized nanodiamonds for quantitative measurement of reducing sugars in the food industry.

Therefore, the present invention has utility in industry field, notably in food industry.

In the description below, the reference into brackets ([1]) refers to the Reference List situated at the end of the text.

### Background of the Invention

Reducing sugars are defined as molecules with an aldehyde group or a free hemiacetal group. They are a major problem in the food industry, especially in the manufacture of potato chips, honey, milk and also wine. Indeed, the presence of reducing sugars, in particular glucose and fructose, in too much quantity in potatoes or honey for example, can generate modifications of the food compound going from an alteration of the organoleptic qualities of the product to harmful consequences on the health of the consumers.

For example, if potatoes intended for the potato chip industry contain too many reducing sugars, then, during the cooking stage, the reducing sugars will produce acrylamide, which is a potentially mutagenic and carcinogenic molecule in human bodies. Moreover, a high level of glucose and fructose in wine leads to a decrease in the acidity of the wine which can itself lead to a sensory alteration. It should be noted that this problem is also found at the honey producers. After pasteurization, if the honey contains a too big quantity of reducing sugars that are glucose and fructose, then it crystallizes quickly, leading to an important granulation.

At present, there are already solutions to measure these reducing sugars. In this purpose, colorimetric methods exist, such as Fehling's liqueur, which is blue in color and contains copper ions and turns brick red in the presence of reducing sugars, but which therefore has the limitation of being a qualitative measurement method. Other methods are polarimetric methods but also enzymatic methods such as the use of glucose oxidase, which is a very specific method, as it only measures the degradation of the initial glucose and not the totality of reducing sugars. Such measurement methods are also qualitative. Moreover, the use of glucose oxidase requires a very precise control of the environment, as this enzyme works optimally at pH=5.6 and at 25°C.

Thus, a need exists of an alternative method, especially to precisely quantify these reducing sugars when the raw material enters the production line or to follow the conservation of the final product. The present invention fulfills these and other needs.

### Description of the invention

After extensive research, the Applicant has managed to develop a solution for quantitative measurement of reducing sugars.

Surprisingly, the Applicant has succeeded in developing a quantitative method of measurement of reducing sugars by labeling with fluorescent nanodiamonds.

Advantageously, the Applicant made nanodiamonds having on their surfaces chemicals or biomolecules that can target reducing sugars. These functionalized nanodiamonds can therefore mark and recognize specifically the reducing sugars. Once the nanodiamonds are functionalized, they bind to reducing sugars and, thanks to the fluorescence property of the nanodiamonds used, a quantitative measurement of reducing sugars is then possible.

One of the advantages of the invention is that the measurement can be the long-term stability of fluorescence signal of nanodiamonds. Another advantage is that the technology is sensitive, accurate and capable of measuring all the reducing sugars, unlike the techniques of prior art mentioned above. Moreover, the invention allows a measurement on the field, point of interest and at the earliest possible time during the harvest of the foodstuff, potatoes for example.

Accordingly, in a first aspect, the present invention provides a method for quantitative measurement of reducing sugars in a sample, comprising the steps of:
(1) Functionalizing nanodiamonds having Nitrogen-Vacancy (NV) centers with ligands able to bind specifically to said reducing sugars,
(2) Bringing said functionalized nanodiamonds into contact with said reducing sugars, under conditions allowing binding between said nanodiamond and said reducing sugars,
(3) Illuminating the NV centers with green light, and
(4) Measuring red fluorescence emitted by the NV centers,
wherein the amount of red fluorescence emitted by the NV centers is proportional to the amount of nanodiamonds bound to the reducing sugars.

"Reducing sugars" refers herein to any reducing sugar, for example at least one sugar selected among glucose, fructose, glyceraldehyde, lactose, arabinose and maltose. Especially, the invention allows to measure several reducing sugars simultaneously or in a distinct way, depending on the functionalization of the nanodiamonds chosen. For example, it may be desirable to measure simultaneously glucose and fructose.

"Nanodiamonds" refers herein to diamonds having nanoscale. Advantageously, the nanodiamonds may have a size included between 50nm and 150nm, or 70nm and 100 nm.

Optionnally, the nanodiamonds used in step (1) may be carboxylated. "Carboxylated" refers herein to a carboxyl group at the surface of nanodiamonds, that are able to be functionalized using carbodiimide chemistry. For example, carboxyl group may be present at the surface of fluorescent nanodiamonds due to chemical treatment.

"Nitrogen-Vacancy (NV) centers" refers herein to defects that are artificially created in nanodiamonds during their preparation. A carbon atom in the diamond's crystal lattice is replaced by a nitrogen atom (defect, N), and a neighboring lattice site is left empty (vacancy, V) to form NV center. These defects exhibit red fluorescence properties under green light excitation. As further explained below, the fluorescence properties of nanodiamonds with NV centers may be processed and quantitatively linked to reducing sugars concentration.

"Functionalizing" refers herein to the linkage of chemicals or biomolecules at the surface of the nanodiamonds. Advantageously, functionalization of nanodiamonds make them able to recognize and link specifically reducing sugars. Advantageously, functionalization of the surface carboxyl groups of nanodiamonds may be realized by any method known in the art for this purpose, as for example described in reference [1] for quantum dots. (Muhammad et al. "Simultaneous determination of insulin and glucose in human serum based on dual emissive fluorescent nano-aptasensor of carbon dots and CdTe/CdS/ZnS quantum dots". Sensors & Actuators: B. Chemical 292 (2019) 321-330 ([1])). For example, in a first step, carboxylated groups on the nanodiamonds may be activated using 1-ethyl-3-(-3-dimethylaminopropyl) carbodiimide hydrochloride (EDC) and N-hydroxysuccinimide (NHS) or Sulfo-NHS. The activated carboxyl groups are made to react with NH₂ terminated ligand or amine part of the ligand able to bind specifically to reducing sugars to form ligand-nanodiamond via amide linkage between the carboxyl and amine groups. When reducing sugar of interest is made to react with ligand functionalized nanodiamonds, it results in the attachment of said reducing sugar to the surface of the nanodiamonds, and thus the presence of reducing sugars can be detected by using nanodiamond as a label.

"Ligands able to bind specifically to reducing sugars" refers herein to chemicals or biomolecules known in the art to link at least one reducing sugar. It may be for example aptamers, which may be DNA or polyclonal antibodies.

For example, nanodiamond may be functionalized with glucose aptamer, in order to recognize and bind glucose. In this purpose, glucose aptamer with sequence 5 ' -NH₂-(CH ₂)₆-AGCTACAGCTAC-3' (SEQ ID NO: 1) can be functionalized onto carboxylated nanodiamond using carbodiimide chemistry. Here, the amine group of the aptamer can be linked to the carboxyl (COOH) groups of the nanodiamonds as explained above. The activated carboxyl groups are made to react with NH₂ terminated glucose aptamer to form aptamer-nanodiamond via amide linkage between the carboxyl and amine groups. When glucose is made to react with aptamer functionalized nanodiamonds, it results in change in the conformation of the aptamer on binding to glucose, and thus the presence of glucose can be detected by using nanodiamond as a label.

In another example, nanodiamond may be functionalized with polyclonal anti-lactose antibodies.

The step (2) of bringing said functionalized nanodiamonds into contact with said reducing sugars may be realized by any method known by the skilled person for this purpose. This can be performed e.g. *in vitro* in a plate pre-functionalized with capture antibodies for a specific reducing sugar, for example by incubating the functionalized nanodiamonds in a medium containing the reducing sugars. Period and conditions of culture may be determined by the skilled person in view of his technical skills.

Illuminating the NV centers with green light may be realized at a wavelength of between 480 and 600 nm. Advantageously, the red fluorescence emitted by NV centers in nanodiamonds is modulated with an electromagnet, in order to improve the limit of detection. The skilled person can choose according his common knowledge in this technical field an optimized modulation frequency and numerical filter, in order to get rid of the continuous component of the autofluorescence. Then, the measurement may be advantageously more sensitive for nanodiamonds fluorescence and then for analyte amount.

Nanodiamonds bound to reducing sugars may be detected by measuring red fluorescence emitted by the NV centers. Red fluorescence may be measured by any mean known in the state of the art, for example with an optical detector. As each functionalized nanodiamond is bound to sugar molecules, the amount of red fluorescence emitted is proportional to the amount of nanodiamonds bound to the reducing sugars, therefore allowing quantitative measurement of reducing sugars.

In an aspect of the invention, the functionalized nanodiamonds may be incorporated into an immunodiagnostic membrane. This immunodiagnostic membrane may be a strip, especially commonly used self-test type. In this purpose, the strip may be placed into a cassette, as those commonly used for these kinds of test, which contains a zone able to receive the drops of sample, and a zone wherein the sample is visible and able to migrate.

The strip may be any strip known in the state of the art for this purpose, as for example a lateral flow strip.

Advantageously, the strip may comprise the areas commonly found on such self-test.

It may be notably a sample drop area (also called "the sample pad"), an area in which the functionalized nanodiamonds are incorporated (also called "the conjugate pad"), a detection area (also called "nitrocellulose pad" or "NC membrane") and an absorbent area (also called "the absorbent pad").

The detection area generally comprises a test line and a control line. The test line contains antibodies able to bind specifically to the reducing sugars linked to the functionalized nanodiamonds. When they arrive on the test line, the functionalized nanodiamonds linked to the reducing sugars are immobilized on the line. The control line contains other antibodies, able to bind to the functionalized nanodiamonds. When they arrive on the control line, the functionalized nanodiamonds not linked to the reducing sugars are immobilized on the line and may be revealed by the fluorescence signal of nanodiamonds.

The absorbent pad contains a highly absorbent material to prevent the leakage of remaining sample crossing the control line from the strip.

Advantageously, the functionalized nanodiamonds, eventually bound to the reducing sugars, migrate by capillarity along the strip to the detection area, after the sample containing reducing sugars is deposited on the sample drop area.

Advantageously, the detection area may be illuminated by a green laser with a wavelength between 480 nm and 600 nm, in order to allow quantitative measurement of reducing sugars. Advantageously, as explained above, the amount of red light emitted is proportional to the amount of nanodiamonds bound to the reducing sugars trapped on the test line of the strip.

In a further aspect, the present invention provides a carboxylated nanodiamond having Nitrogen-Vacancy (NV) centers, which is functionalized with aptamers or with polyclonal antibodies that are able to bind specifically to said reducing sugars, as defined above.

In another aspect, the present invention provides a device comprising a processor and memory, said memory comprising code that, when executed by said processor, causes the device to perform the method of the invention.

When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), Graphical Processing Unit (GPU), read only memory (ROM) for storing software, random access memory (RAM), and non-volatile storage. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

According to the invention, memory may include or store computer program code, and the memory and the computer program code may be configured to, with the processor, cause a network element or network device to perform the necessary tasks. Additionally, the processor, memory and example algorithms, encoded as computer program code, serve as means for providing or causing performance of operations discussed herein.

In another aspect, the present invention provides the use of the method of the invention, or of a functionalized nanodiamond of the invention, for quantitative measurement of reducing sugars in the food industry. Advantageously, the quantitative measurement of reducing sugars may be realized in potatoes, for example a potato juice, milk, honey or wine. For example, if the potato chips industry wishes to differentiate the amount of fructose from the amount of glucose present in its potatoes in order to select a specific potato variety, to obtain fine organoleptic properties (textures, color or another characteristic of the chips) it is possible to functionalize the nanodiamonds with a ligand to specifically recognize glucose and with another ligand to specifically recognize fructose. The same is true for the honey and wine industry.

In another aspect, the present invention provides a method for preparing a carboxylated nanodiamond having Nitrogen-Vacancy (NV) centers, which is functionalized with aptamers that are able to bind specifically to reducing sugars, comprising the steps of:
(A) activating the carboxylated groups on the nanodiamonds using 1-ethyl-3-(-3-dimethylaminopropyl) carbodiimide hydrochloride (EDC) and N-hydroxysuccinimide (NHS) or Sulfo-NHS, and
(B) reacting the activated carboxyl groups with NH₂ terminated aptamer to bind an aptamer to nanodiamond via amide linkage between the carboxyl and amine groups,
thereby obtaining said functionalized nanodiamond.

This invention is further illustrated by the following examples with regard to the annexed drawings that should not be construed as limiting.

### Brief description of the figures

- Figure 1: represents an illustration of: A: functionalization of glucose aptamer on carboxylated functionalized nanodiamonds (FNDs). B: change in conformation of glucose aptamer on glucose binding.
- Figure 2: represents a strip with the different pads: the 'sample pad' (also called "sample drop area" above), the conjugate pad (which is the area in which the functionalized nanodiamonds are incorporated), the nitrocellulose membrane (NC Membrane, also possibly called "detection area" above), and the absorbent pad (also called "absorbent area above).
- Figure 3: represents the reducing sugars (called C), which link to the functionalized nanodiamond and after, the complex functionalized nanodiamond with the molecule of reducing sugar is revealed.
- Figure 4: represents a modulated magnetic field as well as a green laser, to which are subjected the nanodiamonds bound to glucose molecules.

### Examples

### Example 1: fluorescent nanodiamonds (FND) functionalization with glucose aptamer

Glucose aptamer (DNA oligonucleotides) with sequence (5 ' -NH2-(CH 2)6-AGCTACAGCTAC-3') is functionalized onto carboxylated nanodiamond using carbodiimide chemistry.

Here, the amine group of the aptamer is linked to the COOH groups of the carboxylated nanodiamonds.

In the first step, the carboxylated groups on the nanodiamonds are activated using 1-ethyl-3-(-3-dimethylaminopropyl) carbodiimide hydrochloride (EDC) and N-hydroxysuccinimide (NHS) or Sulfo-NHS in a similar way as Muhammad et al. ([1]). The activated carboxyl groups are made to react with NH₂ terminated glucose aptamer to form aptamer-FND via amide linkage between the carboxyl and amine groups.

When analyte of interest, glucose is made to react with aptamer functionalized FNDs, it results in change in the conformation of the aptamer to bind the glucose to the FND, and thus the presence of glucose can be detected by using FND as a label.

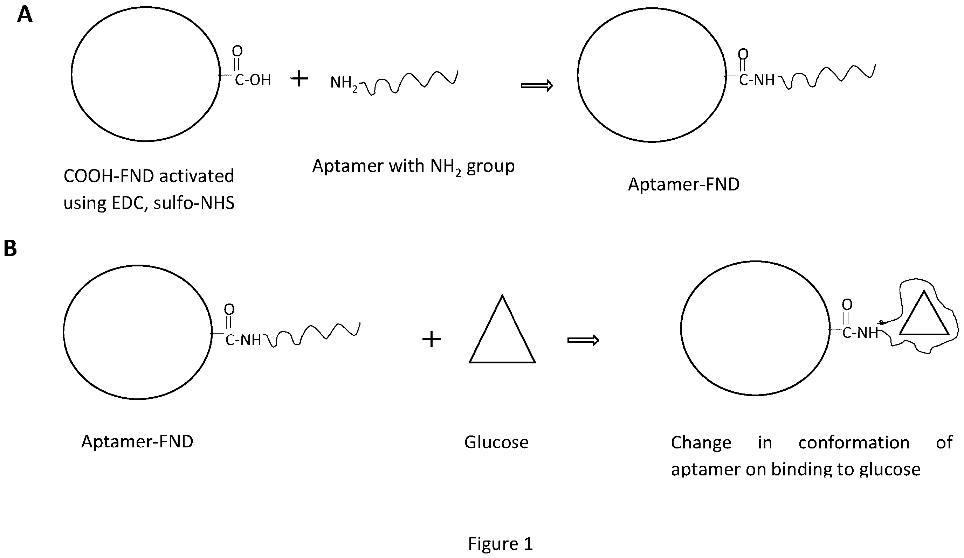

The method of aptamer functionalization and binding of glucose is schematically illustrated in Figure 1.

### Example 2: incorporation of the nanodiamonds previously functionalized into a lateral flow strip

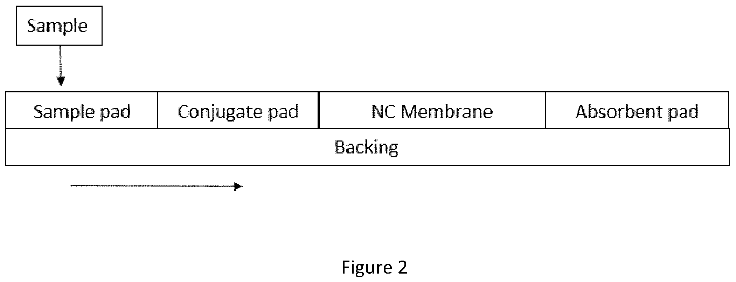

The nanodiamonds previously functionalized in the previous step are incorporated into a lateral flow strip composed of nitrocellulose as shown in Figure 2, on a nitrocellulose membrane (NC Membrane, also possibly called "detection area" above) before the beginning of the analysis.

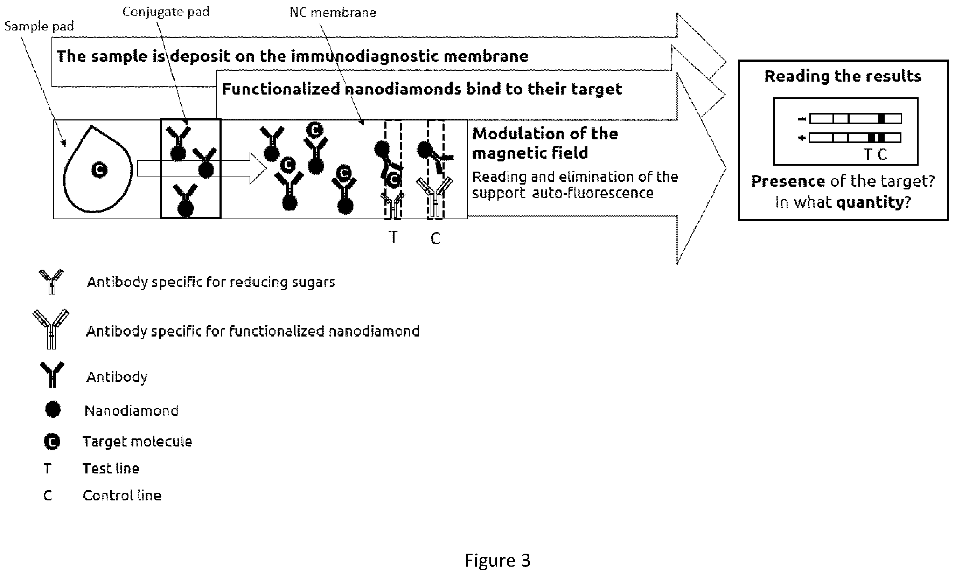

Then, the sample containing reducing sugars is deposited in the first part of the strip called 'sample pad' (also called "sample drop area" above) and migrates along the strip. The reducing sugars couple to the functionalized nanodiamonds present in the conjugate pad (which is the area in which the functionalized nanodiamonds are incorporated). Nanodiamonds coupled to reducing sugars continue to migrate along the strip to the detection area, illustrated by the NC membrane on Figure 2. On this strip, it exists a test line to check the presence of reducing sugar and a control line to validate the migration of functionalized nanodiamonds, as shown in Figure 3. The ligand functionalized nanodiamond on binding to the specific reducing sugar, it migrates along the detection area, and the complex is captured at the test line, which indicates the presence of reducing sugar in the test sample. The antibodies on the control line, are immobilized only to confirm the flow if the flow of the functionalized nanodiamonds worked well, independent of the presence/absence of reducing sugar in the sample.

The absorbent pad, also called "absorbent pad", is also represented.

### Example 3: Quantitative measurement of reducing sugars

To read the result and the amount of reducing sugar in the sample as previously tested with the test strip, the strip under the form of a cassette is put on equipment. Then, the strip receives the laser and the magnetic field.

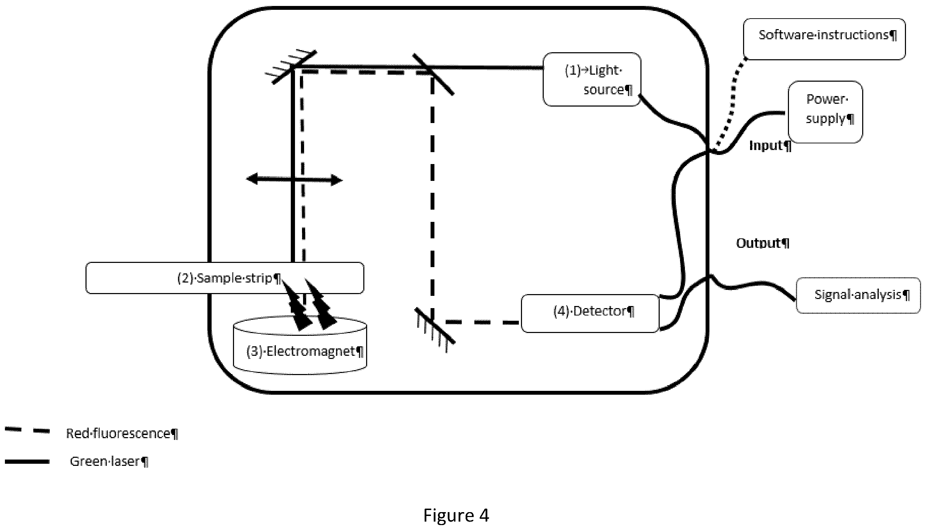

The detection area is illuminated by a green laser with a wavelength between 480 nm and 600 nm. Then, nanodiamonds illuminated by green light, emit red light as shown in Figure 4. To improve the limit of detection, the fluorescence signal is modulated with an electromagnet as shown in this figure. By choosing the right modulation frequency and a numerical filter, it is possible to get rid of the continuous component of the autofluorescence caused by the NC membrane. Then, the measurement is more sensitive for nanodiamonds fluorescence and then for analyte amount.

After that, the result appears on a screen as on figure (Fig.4).

The fluorescence test line of nanodiamonds is linked to the quantity of reducing sugars molecules in the sample.
Indeed, the amount of red light emitted by the test line of the strip is proportional to the amount of nanodiamonds bound to the reducing sugars, as each functionalized nanodiamond binds to sugar molecules.

### Reference List

1. Muhammad et al. Simultaneous determination of insulin and glucose in human serum based on dual emissive fluorescent nano-aptasensor of carbon dots and CdTe/CdS/ZnS quantum dots. Sensors & Actuators: B. Chemical 292 (2019) 321-330.

## Claims

1. A method for quantitative measurement of reducing sugars in a sample, comprising the steps of:
(1) Functionalizing nanodiamonds having Nitrogen-Vacancy (NV) centers with ligands able to bind specifically to said reducing sugars,
(2) Bringing said functionalized nanodiamonds into contact with said reducing sugars, under conditions allowing binding between said nanodiamond and said reducing sugars,
(3) Illuminating the NV centers with green light, and
(4) Measuring red fluorescence emitted by the NV centers,
wherein the amount of red fluorescence emitted by the NV centers is proportional to the amount of nanodiamonds bound to the reducing sugars.

2. A method according to claim 1, wherein said reducing sugars are at least one sugar selected among glucose, fructose, glyceraldehyde, lactose, arabinose and maltose.

3. A method according to any of claim 1 or 2, wherein the functionalized nanodiamonds are incorporated into a strip, for example a lateral flow strip.

4. A method according to claim 3, wherein the strip comprises a sample drop area, an area in which the functionalized nanodiamonds are incorporated, a detection area and an absorbent area.

5. A method according to claim 4, wherein the nanodiamonds bound to the reducing sugars migrate along the strip to the detection area, after the sample containing reducing sugars is deposited on the sample drop area.

6. A method according to any of claims 4 or 5, wherein the detection area comprises a test line and a control line, including respectively test antibodies specific of the reducing sugars and control antibodies specific to functionalized nanodiamonds.

7. A method according to any of claims 4 to 6, wherein the detection area is illuminated by a green laser with a wavelength between 480 nm and 600 nm.

8. A method according to anyone of the preceding claims, wherein the red fluorescence signal is modulated with an electromagnet.

9. A method according to anyone of the preceding claims, wherein the amount of red fluorescence emitted by the strip is proportional to the amount of nanodiamonds bound to the reducing sugars.

10. A method according to anyone of the preceding claims, wherein ligands are selected among aptamer and polyclonal anti-lactose antibody.

11. A method according to claim 10, wherein said aptamer is a glucose aptamer having the sequence 5 ' -NH₂-(CH₂)₆-AGCTACAGCTAC-3' (SEQ ID NO: 1).

12. A carboxylated nanodiamond having Nitrogen-Vacancy (NV) centers, which is functionalized with aptamers or with polyclonal antibodies that are able to bind specifically to reducing sugars, for example for binding glucose aptamer.

13. A device comprising a processor and memory, said memory comprising code that, when executed by said processor, causes the device to perform the method according to any one of the claims 1 to 11.

14. Use of a method as defined in any of claims 1 to 11, or of a functionalized carboxylated nanodiamond as defined in claim 12, for quantitative measurement of reducing sugars in the food industry, for example in potatoes, milk, honey or wine.

15. A method for preparing a carboxylated nanodiamond having Nitrogen-Vacancy (NV) centers, which is functionalized with aptamers that are able to bind specifically to said reducing sugars as defined in claim 12, comprising the steps of:
(A) activating the carboxylated groups on the nanodiamonds using 1-ethyl-3-(-3-dimethylaminopropyl) carbodiimide hydrochloride (EDC) and N-hydroxysuccinimide (NHS) or Sulfo-NHS, and
(B) reacting the activated carboxyl groups with NH₂ terminated aptamer to bind an aptamer to nanodiamond via amide linkage between the carboxyl and amine groups,
thereby obtaining said functionalized nanodiamond.
